# EUROPEAN PATENT SPECIFICATION

(11) **EP 3 996 688 B1**
(45) Date of publication and mention of the grant of the patent: **06.09.2023**
(21) Application number: 20736349.0
(22) Date of filing: 08.07.2020
(51) Int. Cl.: A61K 9/16, A61K 9/20, A61K 9/48, A61K 31/506, A61P 35/00

(54) **PHARMACEUTICAL PREPARATION**
PHARMAZEUTISCHES PRÄPARAT
PRÉPARATION PHARMACEUTIQUE

(30) Priority: 10.07.2019 EP 19185500
(43) Date of publication of application: 18.05.2022
(73) Proprietor: Merck Patent GmbH, 64293 Darmstadt (DE)
(72) Inventor: SCHOCH, Corinna, 68526 Ladenburg (DE); RIEHL, Markus, 64287 Darmstadt (DE); HOOFF, Gero, 68642 Buerstadt (DE); KLEMM, Markus, 64807 Dieburg (DE); SCHMIDT, Carsten, 55131 Mainz (DE); WEIGANDT, Markus, 68259 Mannheim (DE)
(86) International application number: PCT/EP2020/069165
(87) International publication number: WO 2021/005077

(56) References cited:
- WO-A1-2010/078897
- WO-A1-2014/067610
- WO-A1-2015/104042

## Description

The present invention relates to a solid pharmaceutical preparation of 3-(1-{3-[5-(1-Methyl-piperidin-4-ylmethoxy)-pyrimidin-2-yl]-benzyl}-6-oxo-1,6-dihydro-pyridazin-3-yl)-benzonitrile, as well as a method of making same, as well as medical uses thereof.

3-(1-{3-[5-(1-Methyl-piperidin-4-ylmethoxy)-pyrim idin-2-yl]-benzyl}-6-oxo-1,6-dihydro-pyridazin-3-yl)-benzonitrile is disclosed as Example 40 in WO 2009/006959 A1, as one member of a family of pyridazone derivative, which have been found to have valuable pharmacological properties. Specific salts of 3-(1-{3-[5-(1-Methyl-piperidin-4-ylmethoxy)-pyrimidin-2-yl]-benzyl}-6-oxo-1,6-dihydro-pyridazin-3-yl)-benzonitrile are disclosed in WO 2009/007074 A1. It is a potent c-Met inhibitor that inhibit the enzymatic activity of c-Met tyrosine kinase. c-Met is a proto-oncogene that encodes a protein known as hepatocyte growth factor receptor (HGFR) inhibitors. Inhibition of c-Met has been proved as a promising approach in the treatment of cancer alone and in combination with other treatments, including chemo-, radio- and/or immunotherapy.

3-(1-{3-[5-(1-Methyl-piperidin-4-ylmethoxy)-pyrim idin-2-yl]-benzyl}-6-oxo-1,6-dihydro-pyridazin-3-yl)-benzonitrile has a very low solubility in water and biorelevant media. In detail 3-(1-{3-[5-(1-Methyl-piperidin-4-ylmethoxy)-pyrimidin-2-yl]-benzyl}-6-oxo-1,6-dihydro-pyridazin-3-yl)-benozonitrile has a solubility in Fasted Simulated Intestinal Fluid (FaSSIF) of 43 µg/mL and in Fed State Simulated Intestinal Fluid (FeSSIF) of 319 µg/mL. Despite such low solubility rather high doses of far above 100 mg are needed for its use in the therapy. With an estimated efficacious human dose of about 500 mg, 3-(1-{3-[5-(1-Methyl-piperidin-4-ylmethoxy)-pyrimidin-2-yl]-benzyl}-6-oxo-1,6-dihydro-pyridazin-3-yl)-benzonitrile has a dose/solubility ratio of at >10000 and can be classified as BCS IV (Amidon et al., 1995). Accordingly, a pharmaceutical preparation for oral administration must have a high bioavailability to provide the high doses that are needed to achieve the desired therapeutic effect. Otherwise the pharmaceutical preparation would need to have an increased size not usable for its oral administration due to problems with its swallowability.

Micronization of 3-(1-{3-[5-(1-Methyl-piperidin-4-ylmethoxy)-pyrimidin-2-yl]-benzyl}-6-oxo-1,6-dihydro-pyridazin-3-yl)-benzonitrile improves the bioavailability but cause other effects like stickiness and very poor flowability as well as unfavorable compaction properties which are detrimental to the development of a robust formulation that can be produced at an industrial scale (e.g. 50.000 to 100.000 tablets/ batch) as it is required to enable supply of phase III clinical studies as well as market supply.

In addition, 3-(1-{3-[5-(1-Methyl-piperidin-4-ylmethoxy)-pyrimidin-2-yl]-benzyl}-6-oxo-1,6-dihydro-pyridazin-3-yl)-benzonitrile exhibits a high elasticity which is known to be associated with poor compression properties. In fact, 3-(1-{3-[5-(1-Methyl-piperidin-4-ylmethoxy)-pyrimidin-2-yl]-benzyl}-6-oxo-1,6-dihydro-pyridazin-3-yl)-benzonitrile has an elasticity comparable to maize starch, which is known to have poor compaction probabilities due to its elasticity (Lean M. et al., Pharm Dev Technol, 2015; 20(1): 12-21). In the cited reference, a classification is proposed about the properties of different pharmaceutically used excipients and their use for different manufacturing processes. For maize starch, a higher risk for dry granulation techniques is described due to high elastic recovery properties, making this excipient not suitable for this manufacturing process.

Furthermore, it is known in the art that direct compression is normally only feasible for potent drugs where the drug content is less than 30 % of the formulation (Jivraj M. et al., PSTT Vol. 3, No. 2 Feb 2000). As a formulation should deliver 3-(1-{3-[5-(1-Methyl-piperidin-4-ylmethoxy)-pyrimidin-2-yl]-benzyl}-6-oxo-1,6-dihydro-pyridazin-3-yl)-benzonitrile at high doses in the range of about 500 mg, the size of the formulation is limited to allow its swallowability, therefore a drug load above 30 % is necessary.

Alternative approaches to overcome the unfavorable compaction properties of micronized 3-(1-{3-[5-(1-Methyl-piperidin-4-ylmethoxy)-pyrimidin-2-yl]-benzyl}-6-oxo-1,6-dihydro-pyridazin-3-yl)-benzonitrile by using wet granulation techniques that can be used at an industrial scale failed. For example, if micronized 3-(1-{3-[5-(1-Methyl-piperidin-4-ylmethoxy)-pyrimidin-2-yl]-benzyl}-6-oxo-1,6-dihydro-pyridazin-3-yl)-benzonitrile is used in fluid-bed granulation it is blown into the filter as soon as the fluidized bed is set into place so that a pharmaceutical formulation containing such active ingredient cannot be obtained. Manufacturing by means of high-shear granulation proved not successful since the prototypes developed with this manufacturing technique did not show sufficient in-vitro dissolution results.

It was therefore an object of the present invention to provide a pharmaceutical dosage form of 3-(1-{3-[5-(1-Methyl-piperidin-4-ylmethoxy)-pyrimidin-2-yl]-benzyl}-6-oxo-1,6-dihydro-pyridazin-3-yl)-benzonitrile that would provide sufficient bioavailability and which has a high drug load, and a suitable process for its manufacture.

Various attempts to provide a suitable pharmaceutical preparation that provides a bioavailability 3-(1-{3-[5-(1-Methyl-piperidin-4-ylmethoxy)-pyrimidin-2-yl]-benzyl}-6-oxo-1,6-dihydro-pyridazin-3-yl)-benzonitrile in an amount necessary for its use in therapy such as oral solutions, self-microemulsifying drug delivery system SEDDS or SMEDDS failed. SEDDS/ SMEDDS or emulsions cannot be prepared due to the low solubility of 3-(1-{3-[5-(1-Methyl-piperidin-4-ylmethoxy)-pyrimidin-2-yl]-benzyl}-6-oxo-1,6-dihydro-pyridazin-3-yl)-benzonitrile in the tested oils (for example, solubility in neutral oil < 5 mg/mL). Due to the relatively high doses necessary for therapeutic effect (approximately 500 mg) and a maximal dose in a self-emulsifying capsule formulation (SEDDS or SMEDDS) of not more than 5 mg, the patient would have to take 100 capsules to achieve the target dose. Accordingly, this formulation path proved not feasible.

### SUMMARY OF THE INVENTION

The present invention is directed to a solid preparation comprising micronized 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof and a filler, wherein 3-(1-{3-[5-(1-Methyl-piperidin-4-ylmethoxy)-pyrim idin-2-yl]-benzyl}-6-oxo-1,6-dihydro-pyridazin-3-yl)-benzonitrile or its pharmaceutical acceptable salt is present in an amount from 20 to 80 % by weight.

### 3-(1-{3-[5-(1-Methyl-piperidin-4-ylmethoxy)-pyrimidin-2-yl]-benzyl}-6-oxo-1,6-dihydro-pyridazin-3-yl)-benzonitrile is illustrated below:

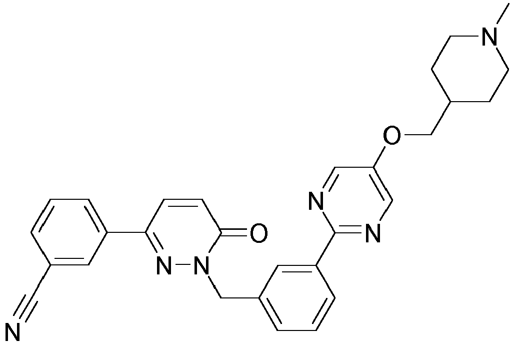

The term "about", as used herein, refers to a numeric value, including, for example, whole numbers, fractions, and percentages, whether or not explicitly indicated. The term "about" generally refers to a range of numerical values (e.g., +/- 1-3% of the recited value) that one of ordinary skill in the art would consider equivalent to the recited value (e.g., having the same function or result). In some instances, the term "about" may include numerical values that are rounded to the nearest significant figure.

As used herein, "a" or "an" shall mean one or more. As used herein when used in conjunction with the word "comprising," the words "a" or "an" mean one or more than one. As used herein "another" means at least a second or more. Furthermore, unless otherwise required by context, singular terms include pluralities and plural terms include the singular.

As used herein, "%" or "percent" shall mean percent by weight (% (w/w)), unless specified otherwise herein.

The present invention further pertains to a pharmaceutical preparation comprising said solid preparation, methods of preparing the solid preparation and methods of preparing the pharmaceutical preparation, as well as the use of the solid preparation respectively pharmaceutical preparation in the treatment of cancer, either alone or in combination with radiotherapy, chemotherapy and/or immunotherapy.

The term "solid preparation", as used herein, refers to a three-dimensional solid pharmaceutical preparation comprising an active pharmaceutical ingredient (API) and at least one pharmaceutically acceptable excipient. Preferably the solid preparation is a compressed mixture of 3-(1-{3-[5-(1-Methyl-piperidin-4-ylmethoxy)-pyrimidin-2-yl]-benzyl}-6-oxo-1,6-dihydro-pyridazin-3-yl)-benzonitrile and one or more pharmaceutically acceptable excipients, for instance selected from a filler and optionally one or more pharmaceutically acceptable excipients. The compressed mixture is obtainable by dry granulation and preferably exists in the form of particles which may have an irregular or regular shape. The solid preparation may be processed to other pharmaceutical preparations such as, for example tablets, but may also be administered to the patient directly without any modification.

Beside the filler one or more further excipients such as a binder, a glidant, a disintegrant and a lubricant may be present in the solid preparation.

The term "micronized", as used herein, refers to particles that have been reduced to micron size. According to an appropriate embodiment micronized 3-(1-{3-[5-(1-Methyl-piperidin-4-ylmethoxy)-pyrimidin-2-yl]-benzyl}-6-oxo-1,6-dihydro-pyridazin-3-yl)-benzonitrile present in the solid preparation have a mean particle size that is characterized by a d50 value in the range from 5 µm to 80 µm, preferably from 5 µm to 50 µm and more preferably from 5 µm to 25 µm. Therefore, the invention is also directed to a solid preparation, wherein 3-(1-{3-[5-(1-Methyl-piperidin-4-ylmethoxy)-pyrimidin-2-yl]-benzyl}-6-oxo-1,6-dihydro-pyridazin-3-yl)-benzonitrile has a mean particle size that is characterized by a d50 value in the range from 5 µm to 80 µm, preferably from 5 µm to 50 µm and more preferably from 5 µm to 25 µm.

The d₅₀ values for 3-(1-{3-[5-(1-Methyl-piperidin-4-ylmethoxy)-pyrimidin-2-yl]-benzyl}-6-oxo-1,6-dihydro-pyridazin-3-yl)-benzonitrile are measured by laser diffraction on a Malvern Mastersizer 2000 (wet method using Hydro 2000S; micro volume tray; sample amount of 100mg; stirrer speed 2200rpm, sonication for 1 min, measuring time of 7.5 s; obscuration of 10-15%). The d₅₀ value referred to herein is the size in micrometers that splits the distribution with half above and half below this diameter. The d50 is the median for a volume distribution and is often also designated Dv50 (or Dv0.5).

Particle sizes of the solid preparations are measured by dynamic image analysis (Retsch CamSizer X2) using a brush and a pin, sample volume is at least 20 mL, Slit width is 4.0 mm, dispersion pressure is 30.0 kPa, no speed adaption. Sizes are defined for corresponding spheres, sample form is defined as cornered particles.

The term "filler" as used herein is an agent increasing the bulk of the pharmaceutical preparation by providing the quantity of material which is needed to form a solid preparation. A filler also serves to create desired flow properties and compression characteristics in the preparation of the solid preparation as well as of solid pharmaceutical preparations such as tablets and capsule fillers. Fillers usable in the present invention may be a sugar alcohol such as sorbitol or mannitol, dulcitol, xylitol or ribitol, preferably sorbitol or mannitol, particular preferably mannitol, a sugar such as glucose, fructose, mannose, lactose, saccharose or maltose, preferably lactose, saccharose or maltose, particular preferably lactose, a starch such as potato starch, rice starch, maize starch or pregelatinized starch. Filler can be present in the solid preparation according to the invention in a proportion of 20 to 80% (w/w), preferably 30 to 70% (w/w), particularly preferably to 40 to 65% (w/w), based on the total weight of the solid formulation.

Beside the filler one or more further excipients such as a binder, a glidant, a disintegrant and a lubricant may be present in the solid preparation.

The solid preparation of the present invention comprises 3-(1-{3-[5-(1-Methyl-piperidin-4-ylmethoxy)-pyrimidin-2-yl]-benzyl}-6-oxo-1,6-dihydro-pyridazin-3-yl)-benzonitrile in an amount from 20 to 80 % by weight based upon the total weight of the solid preparation. According to preferred embodiments 3-(1-{3-[5-(1-Methyl-piperidin-4-ylmethoxy)-pyrimidin-2-yl]-benzyl}-6-oxo-1,6-dihydro-pyridazin-3-yl)-benzonitrile is present in the solid preparation in an amount from 25 to 70 % by weight, more preferred in an amount from 30 to 60 % by weight and most preferred in an amount from 35 to 55 % by weight based upon the total weight of the solid preparation. Therefore, the invention is also directed to the solid preparation wherein 3-(1-{3-[5-(1-Methyl-piperidin-4-ylmethoxy)-pyrimidin-2-yl]-benzyll-6-oxo-1,6-dihydro-pyridazin-3-yl)-benzonitrile is present in an amount from 20 to 80 % by weight, preferably from 25 to 70 % by weight, more preferably in an amount from 30 to 60 % by weight and most preferably in an amount from 35 to 55 % by weight based upon the total weight of the solid preparation.

Any reference to amounts or weights or weight percentages of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or pharmaceutically acceptable salts thereof, shall be taken to refer to the anhydrous free form of 3-(1-{3-[5-(1-Methyl-piperidin-4-ylmethoxy)-pyrimidin-2-yl]-benzyl}-6-oxo-1,6-dihydro-pyridazin-3-yl)-benzonitrile, unless specified otherwise herein.

The solid preparation can comprise 3-(1-{3-[5-(1-Methyl-piperidin-4-ylmethoxy)-pyrimidin-2-yl]-benzyl}-6-oxo-1,6-dihydro-pyridazin-3-yl)-benzonitrile in the form of its free base but also in the form of a pharmaceutical acceptable thereof. The term "pharmaceutically acceptable", as used herein, refers to that which is useful in preparing a pharmaceutical composition that is generally safe, non-toxic, and neither biologically nor otherwise undesirable and includes that which is acceptable for veterinary as well as human pharmaceutical use. The term "pharmaceutically acceptable salt", as used herein, refers to a salt of a 3-(1-{3-[5-(1-Methyl-piperidin-4-ylmethoxy)-pyrimidin-2-yl]-benzyl}-6-oxo-1,6-dihydro-pyridazin-3-yl)-benzonitrile that is pharmaceutically acceptable, as defined herein, and that possess the desired pharmacological activity of the parent 3-(1-{3-[5-(1-Methyl-piperidin-4-ylmethoxy)-pyrimidin-2-yl]-benzyl}-6-oxo-1,6-dihydro-pyridazin-3-yl)-benzonitrile. The term "pharmaceutically acceptable salt" includes all hydrates of the respective salt. Appropriate salts may be acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic, camphorsulfonic acid, citric acid, methanesulfonic acid, p-toluenesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2 naphthalenesulfonic acid, propionic acid, salicylic acid, succinic acid, tartaric acid, trimethylacetic acid, and the like. Especially suitable pharmaceutically acceptable salts of 3-(1-{3-[5-(1-Methyl-piperidin-4-ylmethoxy)-pyrimidin-2-yl]-benzyl}-6-oxo-1,6-dihydro-pyridazin-3-yl)-benzonitrile that may be present in the solid preparation are sulphate, phosphate, mesylate, besylate, tosylate, fumarate, monohydrochloride monohydrate or maleate, preferably monohydrochloride monohydrate. Thus, the invention is also directed to a solid pharmaceutical preparation, wherein 3-(1-{3-[5-(1-Methyl-piperidin-4-ylmethoxy)-pyrimidin-2-yl]-benzyl}-6-oxo-1,6-dihydro-pyridazin-3-yl)-benzonitrile is present in the form of its sulphate, phosphate, mesylate, besylate, tosylate, fumarate, monohydrochloride monohydrate or maleate, preferably monohydrochloride monohydrate.

According to a preferred embodiment of the invention the solid preparation comprises as filler a sugar, a sugar alcohol or dicalcium phosphate. According to an especially preferred embodiment the filler present in the solid preparation is a sugar alcohol, whereby the sugar alcohol is sorbitol and/or mannitol, preferably mannitol.

According to a further preferred embodiment of the invention the solid preparation comprises a binder. Thus, the invention is also directed to a solid preparation, wherein the solid preparation further comprises a binder.

The term "binder", as used herein, refers to an agent that provides cohesion and strength to a solid preparation. Binders which can be employed in the present invention are, for example, polyvinylpyrrolidone, polyvinyl acetate, a vinylpyrrolidone-vinyl acetate copolymer, polyethylene glycol, a starch paste, such as maize starch paste, a cellulose derivative, such as hydroxypropyl methylcellulose, hydroxypropyl cellulose or microcrystalline cellulose, preferably microcrystalline cellulose. Therefore, the present invention is as well directed to a solid pharmaceutical preparation, wherein the binder is polyvinylpyrrolidone, polyvinyl acetate, a vinylpyrrolidone-vinyl acetate copolymer, polyethylene glycol, a starch paste, such as maize starch paste, a cellulose derivative, such as hydroxypropyl methylcellulose, hydroxypropyl cellulose or microcrystalline cellulose, preferably microcrystalline cellulose. Binder can be present in the solid preparation according to the invention in a proportion of 0 to 20% (w/w), preferably 0 to 10% (w/w), particularly preferably to 0 to 5% (w/w), based on the total weight of the solid formulation.

The solid preparation may further comprise a lubricant. The term "lubricant", as used herein, refers to an inactive ingredient used to prevent sticking of ingredients to one another when dry granulated, filled in capsules or compressed to tablets. A lubricant reduces powder sticking to the roll surface of roller compactors and sliding friction of the tableting material and punches in the die during the tableting operation and prevents sticking to the tablet punches. Suitable lubricants are alkaline-earth metal salts of fatty acids, such as magnesium stearate or calcium stearate, fatty acids, such as stearic acid, higher fatty alcohols such as cetyl alcohol or stearyl alhohol, fats such as glyceryl dipalmitostearate, glyceryl distearate, stearin or glyceryl dibehenate, alkaline-earth metal salts of C16-C18 alkyl substituted dicarbonic acids such as sodium stearyl fumarate, hydrated vegetable oils such as hydrated castor oil or hydrated cotton seed oil, or minerals such as talc. Preferred lubricants are sodium stearyl fumarate, esters of glycerol with fatty acids, stearic acid or pharmaceutically acceptable salts of stearic acid and divalent cations, preferably magnesium stearate. Lubricants can be present in the solid preparation according to the invention in a proportion of 0 to 5% (w/w), preferably 0.1 to 2% (w/w), particularly preferably 0.3 to 1% (w/w), most preferably about 0.5% (w/w), based on the total weight of the solid formulation.

The solid preparation may further comprise a disintegrant. The term "disintegrant", as used herein, refers to a compound that expands and dissolves when wet, to cause disintegration of tablets or granulates to break apart and release the active pharmaceutical agent. The disintegrant also functions to ensure that 3-(1-{3-[5-(1-Methyl-piperidin-4-ylmethoxy)-pyrimidin-2-yl]-benzyl}-6-oxo-1,6-dihydro-pyridazin-3-yl)-benzonitrile is in contact with the solvent, such as water. Disintegrants serve to disintegrate tablets or granules etc. and thus enhance dissolution of the solid dosage form upon contact with the liquid dissolution medium. Suitable disintegrants include crospovidone (cross linked polyvinyl N-pyrrolidone), carboxymethylcellulose and salts and derivatives thereof, such as crosslinked derivatives, for instance croscarmellose sodium (cross-linked polymer of carboxymethylcellulose sodium,) sodium carboxymethyl glycolate, sodium starch glycolate, carrageenan, agar, and pectin. Crospovidone and croscarmellose sodium are particularly preferred. Disintegrants are present in the pharmaceutical preparation according to the invention in a proportion of 0 to 10% (w/w), preferably 0.25 to 5% (w/w), particularly preferably 0.5 to 3% (w/w), based on the total weight of the solid formulation.

The solid preparation may further comprise a glidant. The term "glidant", as used herein, refers to an inactive ingredient used as a flow aid that improves the flow characteristics of particulates such as powders or granules. In the present invention flow characteristics of the solid preparation or the mixtures containing the solid preparation during further processing such as encapsulation or tableting. Nonlimiting examples of glidants for use in the present invention include colloidal silicon dioxide (Aerosil 200, Cab-O-Sil), talc, magnesium carbonate, and combinations thereof. Glidants are present in the pharmaceutical preparation according to the invention in a proportion of 0 to 7.5% (w/w), preferably 0 to 5% (w/w), particularly preferably 0 to 3% (w/w), based on the total weight of the solid formulation.

According to an appropriate embodiment of the invention the solid preparation is in the form of particles having a mean particle size that is characterized by a d50 value in the range from 50 µm to 1 mm, preferably from 60 µm to 800 µm and more preferably from 70 to 600 µm. Thus, the invention is also directed to a solid preparation, wherein the solid preparation has a mean particle size that is characterized by a d50 value in the range from 50 µm to 1 mm, preferably from 60 µm to 800 µm and more preferably from 70 to 600 µm.

In order to form a solid preparation dry granulation can be used. The term "dry granulation" or "dry granulating", as used herein, refers specifically to granulation techniques comprising at least a compaction step. In the pharmaceutical industry two dry granulation methods are primarily used, namely slugging and roller compaction, which both can be used to prepare the solid preparation. Dry granulation by slugging comprises a compaction step using a compression machinery which typically contains two steel punches within a steel die cavity. The granules are formed when pressure is exerted on the material particles by the punches in the cavity and typically have about 25 mm diameter by about 10-15 mm thick, but the particular size of the slug is not a limiting factor for the present invention. Dry granulation by using roller compaction comprises a roller compaction step, wherein material particles are compacted between rotating press rolls, and a subsequent milling step to mill the compacted material into granules. In "dry granulation" processes as usable to prepare the solid preparation, typically, no liquids are employed and/or no drying steps are required. The term "granule" itself does not necessarily imply a specific shape, since the final shape of the granule(s) will be controlled by the specific method of preparation.

The present invention also provides a pharmaceutical preparation comprising the solid preparation according to the invention. Accordingly, the present invention is also directed to a pharmaceutical preparation comprising the solid preparation. The solid preparation may be used as pharmaceutical preparation without any modification but can also be processed to other pharmaceutical preparations such as, for example tablets, or filled into sachets or capsules.

Preferably, the pharmaceutical preparation is for oral administration. Therefore, the present invention is also directed to a pharmaceutical preparation, which is a pharmaceutical preparation for oral administration.

More preferably still, the pharmaceutical preparation is an immediate release preparation. Therefore, the present invention is further directed to pharmaceutical preparation, which is an immediate release preparation.

In exemplary embodiments, the pharmaceutical preparation, preferably a tablet, is characterized by a disintegration time of 30 minutes or less, such as 20 minutes or less, preferably 15 minutes or less, and more preferably 10 minutes or less. The disintegration time referred to above is measured in 0.01 N HCI at 37°C in a disintegration apparatus according to USP-NF <701> (USP39-NF34 Page 537; Pharmacopeial Forum: Volume No. 34(1) Page 155) Disintegration: The apparatus consists of a basket-rack assembly, a 1000-mL, low-form beaker for the immersion fluid, a thermostatic arrangement for heating, and a device for raising and lowering the basket in the immersion fluid. The basket-rack assembly moves vertically along its axis and consists of six open-ended transparent tubes; the tubes are held in a vertical position by two plates. Attached to the under surface of the lower plate is a woven stainless steel wire cloth. If specified in the individual monograph, each tube is provided with a cylindrical disk. The disk is made of a suitable transparent plastic material. Place 1 dosage unit in each of the six tubes of the basket and add a disk. Operate the apparatus, using the specified medium as the immersion fluid, maintained at 37 ± 2°. At the end of the time limit or at preset intervals, lift the basket from the fluid, and observe whether the tablets have disintegrated completely.

In a preferred embodiment, the pharmaceutical preparation according to the present invention is a capsule comprising the solid preparation and optionally one or more pharmaceutically acceptable excipients. The capsule itself may be any pharmaceutically acceptable capsule, such as a hard gelatin capsule, but should preferably be easily dissolvable.

In an exemplary embodiment, the pharmaceutical preparation is a capsule, which contains a mixture consisting of 40 to 100% (w/w), for instance at least 50% (w/w), more preferably at least 70, 80, 90, 95 or 99% (w/w) of the solid preparation according to the present invention; and 0 to 60% (w/w), i.e. the remainder (difference to 100% (w/w)) of the mixture, of at least one pharmaceutically acceptable excipient, preferably selected from a filler, a lubricant, a glidant, a disintegrant and an inorganic alkaline metal salt, based upon the total weight of all material contained in the capsule, i.e. the total weight of the capsule minus the weight of the capsule shell.

Inorganic alkaline metal salts, i.e. salts made up of ions of alkaline metals and inorganic acid anions, have relatively recently been found useful for enhancing dissolution and include sodium chloride, sodium sulphate, sodium carbonate, sodium bicarbonate, sodium phosphate, sodium dihydrogen phosphate, potassium chloride, potassium carbonate, and potassium bicarbonate. Sodium chloride is particularly preferred.

A preferred embodiment of the invention is directed to pharmaceutical preparation, which is a capsule, which contains 40 to 100 % (w/w) of the solid preparation; and 0 to 60 % (w/w) of at least one pharmaceutically acceptable excipient, preferably selected from a filler, a glidant, a disintegrant and a lubricant, based upon the total weight of all material contained in the capsule.

As will be shown by way of examples, capsule formulations may comprise, for instance, 100, 99.5, 99, 90, 80, 75, 70, 60 or 50% (w/w) of the solid preparation, or any range enclosed by any combination of those values, based upon the total weight of all material contained in the capsule. The remainder of the filler (difference to 100% (w/w)) is made up by at least one pharmaceutically acceptable excipient, as set out above.

In an exemplary embodiment, the pharmaceutical preparation is a capsule containing a filler comprising:
50 to 100% (w/w) of the solid preparation according to the invention;
0 to 20% (w/w) of disintegrant;
0 to 50% (w/w) of a filler;
0 to 5% (w/w) of a lubricant;
0 to 5% (w/w) of a glidant
0 to 20% (w/w) of an inorganic alkaline metal salt; and
a total of 0 to 20% (w/w) of one or more additional pharmaceutically acceptable excipients, based upon the total weight of the capsule.

Filler may be present in the above exemplary embodiment, for instance, in a range of 5 to 50% (w/w), or a range of 7.5 to 50% (w/w), or a range of 10 to 40% (w/w), for instance.

Inorganic alkaline metal salt is preferably present in the above exemplary embodiment and may be comprised in an amount of 2.5 to 20% (w/w), or 5 to 17.5% (w/w), for instance, or at least 7.5% (w/w), for instance around 10 or 15% (w/w).

In a more preferred embodiment, the pharmaceutical preparation is a tablet, and therefore typically comprises in addition to the pharmaceutically acceptable excipients present in the solid preparation at least one further pharmaceutically acceptable excipient. The at least one additional pharmaceutically acceptable excipient is preferably selected from a filler, a glidant, a disintegrant, a lubricant, an inorganic alkaline metal salt or a combination thereof. Accordingly, the present invention is also directed to a pharmaceutical preparation, which is a tablet and which in addition to the pharmaceutically acceptable excipients present in the solid preparation optionally comprises one or more pharmaceutically acceptable excipient selected from a filler, a disintegrant, a glidant and a lubricant.

In an exemplary embodiment, the pharmaceutical preparation is a tablet comprising the solid preparation and optionally further excipients, which tablet, based upon its total weight, comprises:
i) 20 to 80 % (w/w) of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof;
ii) 10 to 70 % (w/w) of a filler;
iii) 0 to 20 % (w/w) of a binder;
iv) 0 to 20 % (w/w) of disintegrant;
v) 0 to 5 % (w/w) of a lubricant;
vi) 0 to 7,5 % (w/w) of glidant; and
vii) a total of 0 to 20 % (w/w) of one or more additional pharmaceutically acceptable excipients.

The one or more additional pharmaceutically acceptable excipients may include one or more selected from preservatives, antioxidants, sweeteners, flavours, dyes, surfactants, and wicking agents.

Many excipients may exert more than one function, depending on the other components of the pharmaceutical dosage form. For the sake of clarity, in particular in calculating weight percentages, each pharmaceutically acceptable excipient used in a pharmaceutical preparation according to the present invention is preferably associated with one functionality only, i.e. is either regarded as a disintegrant or a lubricant.

In another exemplary embodiment, the pharmaceutical preparation is a tablet comprising the solid preparation and optionally further excipients, which tablet based upon its total weight comprises:
i) 30 to 70 % (w/w) of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof;
ii) 20 to 60 % (w/w) of a filler;
iii) 0 to 10 % (w/w) of a binder;
iv) 0.25 to 10 % (w/w) of disintegrant;
v) 0 to 4 % (w/w) of a lubricant;
vi) 0 to 5 % (w/w) of a glidant; and
vii) a total of 0 to 10 % (w/w) of one or more additional pharmaceutically acceptable excipients.

In a further exemplary embodiment, the pharmaceutical preparation is a tablet comprising the solid preparation and optionally further excipients, which tablet based upon its total weight comprises:
i) 35 to 60 % (w/w) of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof;
ii) 40 to 60 % (w/w) of a filler;
iii) 0 to 5 % (w/w) of a binder;
iv) 0.5 to 5 % (w/w) of disintegrant;
v) 0.25 to 3 % (w/w) of a lubricant;
vi) 0 to 2 % (w/w) of a glidant; and
vii) a total of 0 to 10 % (w/w) of one or more additional pharmaceutically acceptable excipients.

Preferably, in those embodiments, the filler is mannitol or lactose, the binder is microcrystalline cellulose, the disintegrant is selected from crospovidone, carboxymethylcellulose and salts and derivatives thereof, especially croscarmellose sodium, the lubricant is selected from magnesium stearate, calcium stearate and sodium stearyl fumarate and/or the glidant is selected from colloidal silicon dioxide and derivatives thereof. In an especially preferred embodiment the filler is mannitol, the binder microcrystalline cellulose, the disintegrant is crospovidone, the lubricant is magnesium stearate and the glidant is colloidal silicon dioxide.

Preferably, the total of one or more additional pharmaceutically acceptable excipients is 0 to 10% (w/w), 0 to 7.5% (w/w), 0 to 5% (w/w), 0 to 2.5% (w/w) or 0 to 1% (w/w), for instance 0% (w/w).

Of course, the tablet may be coated, to improve taste and/or appearance and/or to protect the tablet from external influences such as moisture. Any coating shall not count towards the total of 100% (w/w) of pharmaceutically active ingredients and drug substance making up the tablets, as listed above. For film-coating, macromolecular substances, such as modified celluloses, including hydroxypropyl methylcellulose (HPMC), polyvinyl alcohol (PVA), polymethacrylates, polyethylene glycols, and zein may be used, for example. The thickness of the coating is preferably less than 200 µm.

The present invention also provides a method for preparing the solid preparation, which comprises dry-granulating, such as slugging and roller compaction, preferably roller compaction. Accordingly, the present invention is also directed to a method for preparing the solid preparation, the method dry granulating, preferably roller compaction.

The term "roller compaction" or "roller compacting" refers to a process in which powders or particles are forced between two counter rotating rolls and pressed into a solid compact or ribbon. Roller compacting can be carried out with any suitable roller compactor known to the skilled person. Suitable roller compactors include, for example, a Fitzpatrick^{®} Chilsonator IR220 roller compactor of the Fitzpatrick Company, USA. The process parameters, especially the roll force, can be readily accomplished by routine experimentation based upon the common general knowledge of the person skilled in the art. Suitable roll force may be, for example, in the range from 2 to 16 kN/cm, more preferably in the range from 4 to 12 kN/cm and most preferably in the range from 4 to 8 kN/cm.

In an exemplary embodiment, the method comprises:
(a) mixing 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof, and a filler and optionally one or more further pharmaceutically acceptable excipient;
(b) dry granulating the mixture prepared by step (a) to form the solid preparation; and
(c) optionally milling.

Preferred pharmaceutical acceptable excipients used in step (a) are selected from a binder, a disintegrant, a lubricant and a glidant. According to a preferred embodiment, dry granulating used in the method is roller compacting.

The solid preparation prepared can be used for the preparation of pharmaceutical preparations such as tablets or capsules. An exemplary method for preparing a pharmaceutical preparation, which is a tablet, comprising the solid preparation, comprises
(a) conducting the method described above to form the solid preparation;
(b) mixing the solid preparation and one or more pharmaceutically acceptable excipients;
(c) tableting the mixture prepared by step (b); and
(d) optionally film coating of the tablets prepared by step (c).

Tableting respectively compressing into tablets can be performed with commonly used eccentric presses or rotary presses.

An exemplary method for preparing a pharmaceutical preparation, which is a capsule, comprising a solid preparation, comprises
(a) conducting the method to form the solid preparation;
(b) optionally mixing the solid preparation and one or more pharmaceutically acceptable excipient and optionally granulating the mixture obtained, preferably by roller compaction;
(c) filling the mixture or granulate prepared by step (b) or the solid preparation prepared by step (a) into capsules.

As set out above in the introductory section, 3-(1-{3-[5-(1-Methyl-piperidin-4-ylmethoxy)-pyrimidin-2-yl]-benzyl}-6-oxo-1,6-dihydro-pyridazin-3-yl)-benzonitrile has been found to exhibit valuable properties as a c-Met tyrosine kinase inhibitor that finds application in the treatment of cancer. It is currently being investigated in clinical trials.

Accordingly, the present invention provides the solid preparation respectively pharmaceutical preparation as described above, for use in the treatment of cancer.

Optionally the treatment of cancer further comprises radiotherapy. Accordingly, the present invention is also directed to the pharmaceutical preparation of the present invention for use in the treatment of cancer optionally together with radiotherapy. Suitable radiotherapy treatments are described in WO 2012/028233 A1 and incorporated by reference herein.

Optionally, in the alternative or in addition to radiotherapy, the treatment of cancer may comprise chemotherapy. Accordingly, the present invention is also directed to the pharmaceutical preparation for use in the treatment of cancer, wherein the treatment further comprises chemotherapy.

Suitable pharmaceutically active ingredients that may be used in chemotherapy in combination with 3-(1-{3-[5-(1-Methyl-piperidin-4-ylmethoxy)-pyrimidin-2-yl]-benzyl}-6-oxo-1,6-dihydro-pyridazin-3-yl)-benzonitrile include cisplatin and etoposide or a combination thereof, to name but one example.

Optionally, in the alternative or in addition to radiotherapy and/or chemotherapy, the treatment of cancer may comprise immunotherapy. Accordingly, the present invention is also directed to the pharmaceutical preparation for use in the treatment of cancer, wherein the treatment further comprises immunotherapy.

Accordingly, the present invention also provides pharmaceutical preparation in accordance with the present invention for use in the treatment of cancer, wherein the treatment comprises administering to the patient said pharmaceutical preparation, optionally in combination with radiotherapy, chemotherapy or immunotherapy or any combination thereof. In an exemplary embodiment, the present invention provides 3-(1-{3-[5-(1-Methyl-piperidin-4-ylmethoxy)-pyrimidin-2-yl]-benzyl}-6-oxo-1,6-dihydro-pyridazin-3-yl)-benzonitrile, or a pharmaceutically acceptable salt thereof in a solid preparation or pharmaceutical preparation according to the present invention, in combination with at least one additional therapeutic agent selected from etoposide and a platin, for use in the treatment of a cancer selected from colon, lung, head and neck, pancreatic, and histological subtypes thereof, in a patient in need thereof.

In the following, the present invention will be described by reference to exemplary embodiments thereof.

### Detailed Description of the Invention

### Brief Description of the Figures

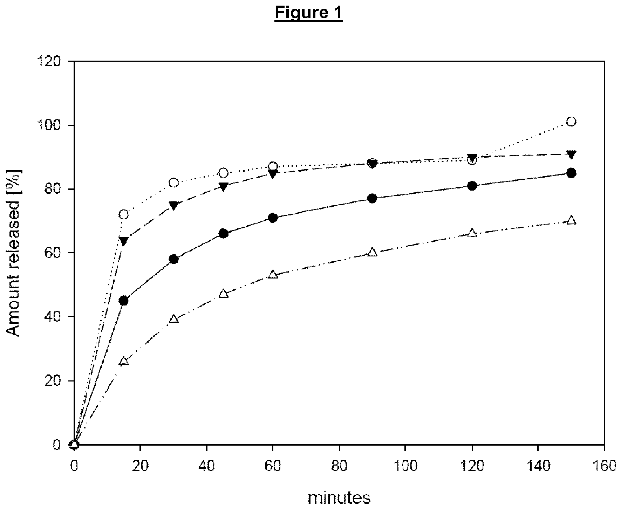

Fig. 1 shows dissolution curves for formulation prototypes (open triangles: Example A; filled circles: Example B; filled triangles: Example C; open circles: Example D), which all contain 3-(1-{3-[5-(1-Methyl-piperidin-4-ylmethoxy)-pyrimidin-2-yl]-benzyl}-6-oxo-1,6-dihydro-pyridazin-3-yl)-benzonitrile. Dissolution conditions are the following: 900 mL acetate buffer of pH=4.5 with 5 mmol/L sodium chloride and 0.1 % Tween 80 using a paddle apparatus with 75 rpm at 37°C.

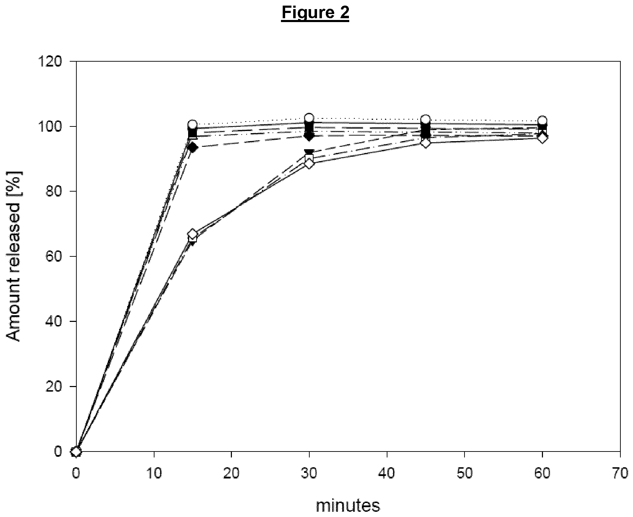

Fig. 2 shows dissolution curves of the examples 12-19 in the dissolution medium acetate buffer pH 4,5 + 3 mmol/ NaCl + 0,1% Tween showing satisfying dissolution. Example 12: filled circles; Example 13: open circles; Example 14: filled triangles; Example 15: open triangles; Example 16: filled squares; Example 17: open squares; filled rhombus: Example 18; open rhombus: Example 19.

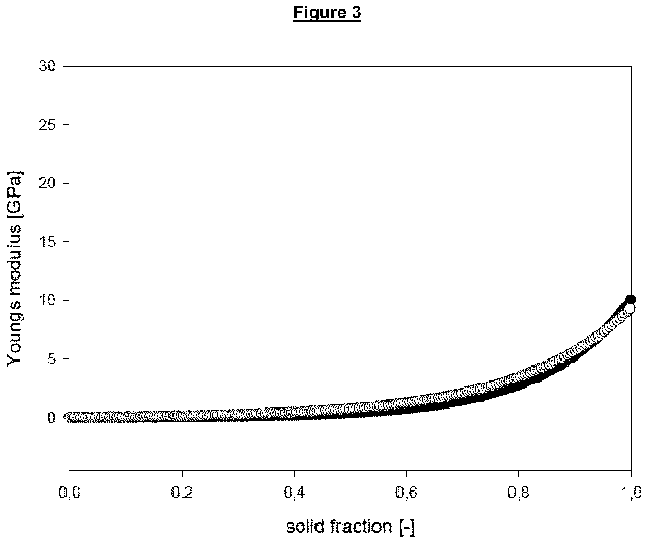

Fig. 3 shows the comparison of 3-(1-{3-[5-(1-Methyl-piperidin-4-ylmethoxy)-pyrimidin-2-yl]-benzyl}-6-oxo-1,6-dihydro-pyridazin-3-yl)-benzonitrile (black dots) with corn starch (open dots) regarding its elastic properties.

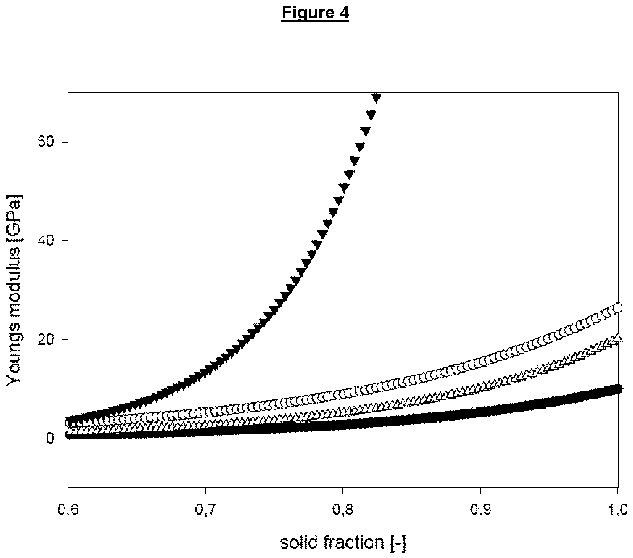

Fig. 4. shows the comparison of 3-(1-{3-[5-(1-Methyl-piperidin-4-ylmethoxy)-pyrimidin-2-yl]-benzyl}-6-oxo-1,6-dihydro-pyridazin-3-yl)-benzonitrile with different pharmaceutically used fillers. Black triangles: dicalcium phosphate, open circles: Mannitol, open triangles: Lactose, black dots: 3-(1-{3-[5-(1-Methyl-piperidin-4-ylmethoxy)-pyrimidin-2-yl]-benzyl}-6-oxo-1,6-dihydro-pyridazin-3-yl)-benzonitrile.

### Preformulation examples

### Examples assessing wet granulation techniques

### EXAMPLE A)

Example A (open triangles in Figure 1) containing 3-(1-{3-[5-(1-Methyl-piperidin-4-ylmethoxy)-pyrimidin-2-yl]-benzyl}-6-oxo-1,6-dihydro-pyridazin-3-yl)-benzonitrile (30.99%), lactose (32.39%), microcrystalline cellulose type 101 (23.00%), povidone 25 (3.76%), Crospovidone (3.76%) are manufactured by a high-shear granulation process. Afterwards, the resulting granules are sieved over 1.0 mm sieve and subsequently blended with Crospovidone (2.35%), povidone 25 (1.41%), magnesium stearate (0.94%), talcum (0.94%) and silicon dioxide (0.47%) and processed to tablets on a single punch press with a resistance to crushing of approx. 125 N, a disintegration time of <10 minutes and a total weight of approx. 645 mg.

### EXAMPLE B)

Example B (filled circles in Figure 1) containing 3-(1-{3-[5-(1-Methyl-piperidin-4-ylmethoxy)-pyrimidin-2-yl]-benzyl}-6-oxo-1,6-dihydro-pyridazin-3-yl)-benzonitrile (77.29%) and starch 1500 (19.32%) has been manufactured by a high-shear granulation process. Afterwards, the resulting granules were sieved over 1.0 mm sieve and subsequently blended with carboxymethyl starch sodium (1.93%), magnesium stearate (0.97%) and silicon dioxide (0.48%) and processed to tablets on a single punch press with a resistance to crushing of approx. 144 N, a disintegration time of <8 minutes and a total weight of approx. 259 mg.

### EXAMPLE C)

Example C (filled triangles in Figure 1) containing 3-(1-{3-[5-(1-Methyl-piperidin-4-ylmethoxy)-pyrimidin-2-yl]-benzyl}-6-oxo-1,6-dihydro-pyridazin-3-yl)-benzonitrile (26.50 %), lactose (4.41%), Hypromellose (1.11%) and calcium phosphate dihydrate (53.01 %) has been manufactured by fluid bed granulation process a person skilled in the art would choose. Afterwards, the resulting granules were sieved over 0.8 mm sieve and subsequently blended with pregelatinized starch (9.89%), magnesium stearate (0.99%), carboxymethyl starch sodium (2.47%) and silicon dioxide (0.49%) and processed to tablets on a single punch press. Afterwards, the tablets were coated using a commercially available preformulated film-coating mixture based on polyvinyl alcohol, with the coating being present to 1.13% in the whole formulation. Resulting tablets showed a resistance to crushing of approx. 159 N, a disintegration time of <6 minutes and a total weight of approx. 755 mg.

### EXAMPLE D)

Example D (open circles in Figure 1) containing 3-(1-{3-[5-(1-Methyl-piperidin-4-ylmethoxy)-pyrimidin-2-yl]-benzyl}-6-oxo-1,6-dihydro-pyridazin-3-yl)-benzonitrile (26.66 %), lactose (4.44%), Hypromellose (1.11%) and calcium phosphate anhydrous (53.31 %) has been manufactured by fluid bed granulation process a person skilled in the art would choose.

Afterwards, the resulting granules were sieved over 0.8 mm sieve and subsequently blended with starch 1500 (9.89%), magnesium stearate (0.99%), carboxymethyl starch sodium (1.98%) and silicon dioxide (0.49%) and processed to tablets on a single punch press to tablet cores of approx. 741.8 mg.

Afterwards, the tablets were coated using a commercially available preformulated film-coating mixture based on polyvinylalcohol, with the coating being present to 1.13% in the whole formulation. Resulting tablets showed a resistance to crushing of approx. 159 N, a disintegration time of <6 minutes and a total weight of approx. 750 mg.

As can be seen from Figure 1 the tablets that are manufactured with granulates that are prepared by high shear granulation (Examples A and B) do not show satisfying in-vitro release properties. Further, tablets that are manufactured with granulates that are prepared by fluid bed granulation (Examples C and D) show better in-vitro release properties than the high shear granulation prototypes (Examples A and B) but they are limited in their maximum achievable drug load.

### Examples assessing the material property Young's modulus

Young's modulus is assessed as an indicator for material stiffness (the higher Young's modulus, the more rigid a substance is) whereby its measurement is done in dependence of the solid fraction, which is complementary to porosity (i.e. solid fraction = 1 - porosity fraction). With this, a solid fraction of 1 indicates no porosity, i.e. no air entrapped in the solid phase. Pharmaceutical relevant solid fractions usually range from 0.75 to 0.85. Porosities have been determined by nitrogen pycnometry.

The measurements to determine the Young's modulus are conducted on a commercially available instrumented single punch press (Romaco Kilian StylOne system) with ultrasound-assisted measurement punches. For this purpose, neat substances are compacted between the upper and the lower punch, leading to densification of the material. The ultrasound velocity in the sample in dependence of the degree of densification is recorded and used for calculation of Youngs modulus of the specific substance.

For this investigation, following neat materials are selected:

| Example | Material |
|---|---|
| E | 3-(1-{3-[5-(1-Methyl-piperidin-4-ylmethoxy)-pyrimidin-2-yl]-benzyl}-6-oxo-1,6-dihydro-pyridazin-3-yl)-benzonitrile |
| F | Corn starch |
| G | dicalcium phosphate |
| H | Mannitol |
| I | Lactose |

As can be seen in Figure 3, 3-(1-{3-[5-(1-Methyl-piperidin-4-ylmethoxy)-pyrimidin-2-yl]-benzyl}-6-oxo-1,6-dihydro-pyridazin-3-yl)-benzonitrile (black dots) shows similar properties as corn starch (open dots), a substance which is known for its high elasticity and therewith unfavorable compression properties. Furthermore, Figure 4 shows that 3-(1-{3-[5-(1-Methyl-piperidin-4-ylmethoxy)-pyrim idin-2-yl]-benzyl}-6-oxo-1,6-dihydro-pyridazin-3-yl)-benzonitrile has distinct lower Young's moduli compared to widely used pharmaceutical fillers (dicalcium phosphate, mannitol and lactose),

### Formulation examples

### EXAMPLE E

### Exemplary solid preparation formulations

D₅₀ values are recorded as described above and are in the range of 70 - 530 µm.

Bulk densities are determined using a 100 mL beaker according to DIN 53468.

### EXAMPLE 1:

The ingredients are weighed (batch size of 103.2 kg) and sieved through a 1.0 mm sieve. The blend is produced by mixing all ingredients except magnesium stearate in a commercially available bin blender (e.g. Servolift) for 15 min with 12 rpm. The magnesium stearate is added afterwards and the whole mixture is blended again for 5 min with 12 rpm. The mixture is transferred afterwards to a roller compactor for manufacturing of the solid preparation. The roller compactor (Gerteis Macropactor) is run with the following settings: Compaction force 12 kN/cm, gap width 2.5 mm, roll speed 3.0 rpm. The resulting granules are sieved through a 0.8 mm sieve.

### EXAMPLE 2:

The ingredients are weighed (batch size of 2.4 kg) and sieved through a 1.0 mm sieve except for magnesium stearate. The blend is produced by mixing all ingredients except magnesium stearate in a commercially available Turbula T50A blender for 15 min with. The magnesium stearate is sieved over 0.5 mm and is added afterwards to the mixture, which is then blended again for 5 min. The mixture is transferred afterwards to a roller compactor for manufacturing of the solid preparation. The roller compactor (Alexanderwerk WP120P) is run with the following settings: Compaction force 4.0 kN/cm, gap width 1.0 mm, roll speed 4.0 rpm. The resulting granules are sieved through a 1.0 mm sieve.

### EXAMPLES 3-8:

The ingredients are weighed (batch size of 1.0 kg) and sieved through a 1.0 mm sieve. The blend is produced by mixing all ingredients in a commercially available Servolift bin blender for 15 min with 12 rpm. The mixture is transferred afterwards to a roller compactor for manufacturing of the solid preparation. The roller compactor (Gerteis Minipactor) is run with the following settings: Compaction force 3.0 kN/cm, gap width 3.0 mm, roll speed 3.0 rpm. The resulting granules are sieved through a 1.0 mm sieve.

### EXAMPLE 9:

The ingredients are weighed (batch size of 33.2 kg) and sieved through a 1.0 mm sieve. The blend is produced by mixing all ingredients in a commercially available Servolift bin blender for 15 min with 12 rpm. The mixture is transferred afterwards to a roller compactor for manufacturing of the solid preparation. The roller compactor (Gerteis Macropactor) is run with the following settings: Compaction force 4.5 kN/cm, gap width 3.0 mm, roll speed 3.0 rpm. The resulting granules are sieved through a 0.8 mm sieve.

| Solid preparation # | Composition | % (w/w) | Bulk density [g/cm³] |
|---|---|---|---|
| 1 | Solid preparation consisting of | | 0.63 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 52.08 | |
| | Mannitol (Parteck M100) | 44.42 | |
| | Silicon dioxide (Aerosil 200) | 2.00 | |
| | Crospovidone (Kollidon CL SF) | 1.00 | |
| | Mg.-stearate | 0.50 | |
| 2 | Solid preparation consisting of | | 0.65 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 70.0 | |
| | Mannitol (Parteck M100) | 28.0 | |
| | Silicon dioxide (Aerosil 200) | 0.5 | |
| | Crospovidone (Kollidon CL SF) | 1.0 | |
| | Mg.-stearate | 0.5 | |
| 3 | Solid preparation consisting of | | 0.60 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 31.6 | |
| | Mannitol (Parteck M100) | 66.9 | |
| | Crospovidone (Kollidon CL SF) | 1.0 | |
| | Mg.-stearate | 0.5 | |
| 4 | Solid preparation consisting of | | 0.61 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 31.6 | |
| | Lactose (SuperTab 30GR) | 66.9 | |
| | Crospovidone (Kollidon CL SF) | 1.0 | |
| | Mg.-stearate | 0.5 | |
| 5 | Solid preparation consisting of | | 0.59 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 31.6 | |
| | Isomalt (Galeniq IQ 721) | 66.9 | |
| | Crospovidone (Kollidon CL SF) | 1.0 | |
| | Mg.-stearate | 0.5 | |
| 6 | Solid preparation consisting of | | 0.59 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 37.5 | |
| | Mannitol (Parteck M100) | 56.0 | |
| | Crospovidone (Kollidon CL SF) | 1.0 | |
| | Mg.-stearate | 0.5 | |
| | Microcrystalline Cellulose (Type 101) | 5.0 | |
| 7 | Solid preparation consisting of | | 0.63 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 37.5 | |
| | Lactose (SuperTab 30GR) | 56.0 | |
| | Crospovidone (Kollidon CL SF) | 1.0 | |
| | Mg.-stearate | 0.5 | |
| | Microcrystalline Cellulose (Type 101) | 5.0 | |
| 8 | Solid preparation consisting of | | 0.61 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 37.5 | |
| | Isomalt (Galeniq IQ 721) | 56.0 | |
| | Crospovidone (Kollidon CL SF) | 1.0 | |
| | Mg.-stearate | 0.5 | |
| | Microcrystalline Cellulose (Type 101) | 5.0 | |
| 9 | Solid preparation consisting of | | 0.56 |
| | 3-(1-{3-[5-(1-Methyl-piperidin-4-ylmethoxy)-pyrimidin-2-yl]-benzyl}-6-oxo-1,6-dihydro-pyridazin-3-yl)-benzonitrile | 37.5 | |
| | Mannitol (Parteck M100) | 55.8 | |
| | Crospovidone (Kollidon CL SF) | 1.2 | |
| | Microcrystalline Cellulose (Type 101) | 5.0 | |
| | Mg.-stearate | 0.5 | |

### Exemplary tablet formulations

Disintegration and friability test are described in the European Pharmacopoeia, Version 9.8, sections 2.9.1 (Disintegration) and section 2.9.7 (Friability of uncoated tablets).

### EXAMPLE 10:

The solid preparation from Example 1 is blended for 15 min with the Crospovidone. The magnesium stearate is added afterwards and the whole mixture is blended again for 5 min with 12 rpm. The whole mixture is tableted with a rotary tablet press, utilizing 18.8 x 9.2 mm punches, a pre-compression force of 1.6 kN and a main compression force of 17.1 kN at a tableting speed of 20000 units/hours.

### EXAMPLE 11:

The solid preparation from Example 2 is blended for 10 min with the ingredients. The whole mixture is tableted with a single punch press, utilizing 18 x 8 mm punches and compression force of 12 kN at a tableting speed of 1860 units/hours. Values for disintegration time and friability are for a resistance to crushing of 100 N.

### EXAMPLE 12:

The solid preparation from Example 3 is blended for 15 min at 12 rpm with all ingredients. The whole mixture is tableted with a single punch press utilizing 19 x 9 mm punches and compression force of 15 kN at a tableting speed of 1500 units/hours. Values for disintegration time and friability are for a resistance to crushing of 150 N.

### EXAMPLE 13:

The solid preparation from Example 4 is blended for 15 min at 12 rpm with all ingredients. The whole mixture is tableted with a single punch press utilizing 19 x 9 mm punches and compression force of 21 kN at a tableting speed of 2460 units/hours. Values for disintegration time and friability are for a resistance to crushing of 110 N.

### EXAMPLE 14:

The solid preparation from Example 5 is blended for 15 min at 12 rpm with all ingredients. The whole mixture is tableted with a single punch press utilizing 19 x 9 mm punches and compression force of 17 kN at a tableting speed of 2520 units/hours. Values for disintegration time and friability are for a resistance to crushing of 160 N.

### EXAMPLE 15:

The solid preparation from Example 6 is blended for 15 min at 12 rpm with all ingredients. The whole mixture is tableted with a single punch press utilizing 19 x 9 mm punches and compression force of 17 kN at a tableting speed of 2460 units/hours. Values for disintegration time and friability are for a resistance to crushing of 150 N.

### EXAMPLE 16:

The solid preparation from Example 7 is blended for 15 min at 12 rpm with all ingredients. The whole mixture is tableted with a single punch press utilizing 19 x 9 mm punches and compression force of 17 kN at a tableting speed of 2460 units/hours. Values for disintegration time and friability are for a resistance to crushing of 110 N.

### EXAMPLE 17:

The solid preparation from Example 8 is blended for 15 min at 12 rpm with all ingredients. The whole mixture is tableted with a single punch press utilizing 19 x 9 mm punches and compression force of 17 kN at a tableting speed of 2460 units/hours. Values for disintegration time and friability are for a resistance to crushing of 150 N.

### EXAMPLE 18:

The solid preparation from Example 6 is blended for 15 min at 12 rpm with all ingredients. The whole mixture is tableted with a single punch press utilizing 19 x 9 mm punches and compression force of 15 kN at a tableting speed of 2460 units/hours. Values for disintegration time and friability are for a resistance to crushing of 165 N.

### EXAMPLE 19:

The solid preparation from Example 8 is blended for 15 min at 12 rpm with all ingredients. The whole mixture is tableted with a single punch press utilizing 19 x 9 mm punches and compression force of 15 kN at a tableting speed of 2460 units/hours. Values for disintegration time and friability are for a resistance to crushing of 170 N.

### EXAMPLE 20:

The solid preparation from Example 7 is blended for 15 min at 12 rpm with all ingredients. The whole mixture is tableted with a single punch press utilizing 19 x 9 mm punches and compression force of 17 kN at a tableting speed of 2460 units/hours. Values for disintegration time and friability are for a resistance to crushing of 150 N.

### EXAMPLE 21:

The solid preparation from Example 9 is blended for 15 min at 12 rpm with all ingredients. The whole mixture is tableted with a rotary tablet press, utilizing 18 x 9 mm punches, a pre-compression force of 5.0 kN and a main compression force of 13.0 kN at a tableting speed of 30000 units/hours.

| Example/ Formulation # | Composition | % (w/w) | Disinte gration time [s] | Friability [%] |
|---|---|---|---|---|
| 10 | Solid preparation as listed in example #1 | 96.00 | 64 | 0.16 |
| | Crospovidone (Kollidon CL SF) | 2.00 | | |
| | Mg.-stearate | 2.00 | | |
| | Solid preparation compressed to tablets and subsequently coated | | | |
| 11 | Solid preparation as listed in example #2 | 98.5 | 94 | 0.28 |
| | Silicon dioxide (Aerosil 200) | 0.5 | | |
| | Mg.-stearate | 1.0 | | |
| | Solid preparation compressed to tablets, potentially coated | | | |
| 12 | Solid preparation as listed in example #3 | 95.0 | 51 | 0.11 |
| | Crospovidone (Kollidon CL SF) | 2.0 | | |
| | Mg.-stearate | 2.0 | | |
| | Silicon dioxide (Aerosil 200) | 1.0 | | |
| 13 | Solid preparation as listed in example #4 | 95.0 | 42 | 0.20 |
| | Crospovidone (Kollidon CL SF) | 2.0 | | |
| | Mg.-stearate | 2.0 | | |
| | Silicon dioxide (Aerosil 200) | 1.0 | | |
| 14 | Solid preparation as listed in example #5 | 95.0 | 337 | 0.13 |
| | Crospovidone (Kollidon CL SF) | 2.0 | | |
| | Mg.-stearate | 2.0 | | |
| | Silicon dioxide (Aerosil 200) | 1.0 | | |
| 15 | Solid preparation as listed in example #6 | 95.0 | 49 | 0.13 |
| | Crospovidone (Kollidon CL SF) | 2.0 | | |
| | Mg.-stearate | 2.0 | | |
| | Silicon dioxide (Aerosil 200) | 1.0 | | |
| 16 | Solid preparation as listed in example #7 | 95.0 | 41 | 0.22 |
| | Crospovidone (Kollidon CL SF) | 2.0 | | |
| | Mg.-stearate | 2.0 | | |
| | Silicon dioxide (Aerosil 200) | 1.0 | | |
| 17 | Solid preparation as listed in example #8 | 95.0 | 302 | 0.13 |
| | Crospovidone (Kollidon CL SF) | 2.0 | | |
| | Mg.-stearate | 2.0 | | |
| | Silicon dioxide (Aerosil 200) | 1.0 | | |
| 18 | Solid preparation as listed in example #6 | 80.0 | 66 | 0.12 |
| | Mannitol (Parteck M200) | 10.0 | | |
| | Crospovidone (Kollidon CL SF) | 2.0 | | |
| | Mg.-stearate | 2.0 | | |
| | Silicon dioxide (Aerosil 200) | 1.0 | | |
| | Microcrystalline Cellulose (Type 102) | 5.0 | | |
| 19 | Solid preparation as listed in example #8 | 80.0 | 345 | 0.11 |
| | Isomalt (Galeniq IQ 721) | 10.0 | | |
| | Crospovidone (Kollidon CL SF) | 2.0 | | |
| | Mg.-stearate | 2.0 | | |
| | Silicon dioxide (Aerosil 200) | 1.0 | | |
| | Microcrystalline Cellulose (Type 102) | 5.0 | | |
| 20 | Solid preparation as listed in example #7 | 80.0 | n.a. | n.a. |
| | Lactose (Tablettose 100) | 10.0 | | |
| | Crospovidone (Kollidon CL SF) | 2.0 | | |
| | Mg.-stearate | 2.0 | | |
| | Silicon dioxide (Aerosil 200) | 1.0 | | |
| | Microcrystalline Cellulose (Type 102) | 5.0 | | |
| 21 | Solid preparation as listed in example #9 | 85.0 | 80 | 0.08 |
| | Mannitol (Parteck M 200) | 10.0 | | |
| | Crospovidone (Kollidon CL SF) | 2.0 | | |
| | Silicon dioxide (Aerosil 200) | 1.0 | | |
| | Mg.-stearate | 2.0 | | |

### Exemplary capsule formulations

Disintegration test is described in the European Pharmacopoeia, Version 9.8, sections 2.9.1 (Disintegration).

### EXAMPLE 22: Exemplary capsule formulations

HPMC capsules containing the different solid preparations from examples 1-9 are prepared by mixing such preparations with the further excipients as depicted below and filling such mixtures into the capsule shells. The disintegration of the capsule formulations is below 9 minutes.

| Example/ Formulation # | Composition | % (w/w) |
|---|---|---|
| 22 | Solid preparation as described in the examples 1-9 | 98.75 |
| | Silicon dioxide (Aerosil 200) | 1 |
| | Magnesium stearate | 0.25 |

## Claims

1. Solid preparation comprising micronized 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof and a filler, wherein 3-(1-{3-[5-(1-Methyl-piperidin-4-ylmethoxy)-pyrimidin-2-yl]-benzyl}-6-oxo-1 ,6-dihydro-pyridazin-3-yl)-benzonitrile or its pharmaceutical acceptable salt is present from 20 to 80 % (w/w) based upon the total weight of the solid preparation.

2. A solid preparation according to Claim 1, wherein 3-(1-{3-[5-(1-Methyl-piperidin-4-ylmethoxy)-pyrimidin-2-yl]-benzyl}-6-oxo-1,6-dihydro-pyridazin-3-yl)-benzonitrile is present in the form of its sulphate, phosphate, mesylate, besylate, tosylate, fumarate, monohydrochloride monohydrate or maleate, preferably monohydrochloride monohydrate.

3. A solid preparation according to Claim 1 or 2, wherein 3-(1-{3-[5-(1-Methyl-piperidin-4-ylmethoxy)-pyrim idin-2-yl]-benzyl}-6-oxo-1,6-dihydro-pyridazin-3-yl)-benzonitrile has a mean particle size that is **characterized by** a d50 value in the range from 5 µm to 80 µm, preferably from 5 µm to 50 µm and more preferably from 5 µm to 25 µm.

4. A solid preparation according to one or more of Claims 1 to 3, wherein the filler is a sugar, a sugar alcohol or dicalcium phosphate.

5. A solid preparation according to Claim 4, wherein the filler is a sugar alcohol, whereby the sugar alcohol is sorbitol and/or mannitol, preferably mannitol.

6. A solid preparation according to one or more of Claims 1 to 5, wherein the solid preparation further comprises a binder.

7. A solid preparation according to Claim 6, wherein the binder is polyvinylpyrrolidone, polyvinyl acetate, a vinylpyrrolidone-vinyl acetate copolymer, polyethylene glycol, a starch paste such as maize starch paste, or a cellulose derivative such as hydroxypropyl methylcellulose, hydroxypropyl cellulose or microcrystalline cellulose, preferably microcrystalline cellulose.

8. A solid preparation according to one or more of Claims 1 to 7, wherein the solid formulation further comprises a lubricant.

9. A solid preparation according to Claim 8, wherein the lubricant is sodium stearyl fumarate, esters of glycerol with fatty acids, stearic acid, or pharmaceutically acceptable salts of stearic acid and divalent cations, preferably magnesium stearate.

10. A solid preparation according to one or more of Claims 1 to 9, wherein the solid preparation has a mean particle size that is **characterized by** a d50 value in the range from 50 µm to 1 mm, preferably from 60 µm to 800 µm and more preferably from 70 to 600 µm.

11. A pharmaceutical preparation comprising the solid preparation according to one or more of Claims 1 to 10.

12. A pharmaceutical preparation according to Claim 11, which is a pharmaceutical preparation for oral administration.

13. A pharmaceutical preparation according to Claim 11 or 12, which is an immediate release preparation.

14. A pharmaceutical preparation according to one or more of Claims 11 to 13, which is a capsule comprising the solid preparation and optionally one or more pharmaceutically acceptable excipients.

15. A pharmaceutical preparation according to Claim 14, which is a capsule, which contains 40 to 100 % (w/w) of the solid preparation according to any of claims 1 to 10; and 0 to 60 % (w/w) of at least one pharmaceutically acceptable excipient, preferably selected from a filler, a glidant, a disintegrant and a lubricant, based upon the total weight of all material contained in the capsule.

16. A pharmaceutical preparation according to one or more of Claims 11 to 13, which is a tablet and which in addition to the pharmaceutically acceptable excipients present in the solid preparation optionally comprises one or more pharmaceutically acceptable excipient selected from a filler, a disintegrant, a glidant and a lubricant.

17. A pharmaceutical preparation according to Claim 16, which is a tablet comprising the solid preparation according to any of claims 1 to 10 and optionally further excipients, which tablet, based upon its total weight, comprises:
i) 20 to 80 % (w/w) of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof;
ii) 10 to 70 % (w/w) of a filler;
iii) 0 to 20 % (w/w) of a binder;
iv) 0 to 20 % (w/w) of disintegrant;
v) 0 to 5 % (w/w) of a lubricant;
vi) 0 to 7,5 % (w/w) of glidant; and
vii) a total of 0 to 20 % (w/w) of one or more additional pharmaceutically acceptable excipients.

18. A pharmaceutical preparation according to Claim 16 or 17, which is a tablet comprising the solid preparation according to any of claims 1 to 10 and optionally further excipients, which tablet based upon its total weight comprises:
i) 30 to 70 % (w/w) of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof;
ii) 20 to 60 % (w/w) of a filler;
iii) 0 to 10 % (w/w) of a binder;
iv) 0.25 to 10 % (w/w) of disintegrant;
v) 0 to 4 % (w/w) of a lubricant;
vi) 0 to 5 % (w/w) of a glidant; and
vii) a total of 0 to 10 % (w/w) of one or more additional pharmaceutically acceptable excipients.

19. A pharmaceutical preparation according to Claim 16, 17 or 18, which is a tablet comprising:
i) 35 to 60 % (w/w) of 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof;
ii) 40 to 60 % (w/w) of a filler;
iii) 0 to 5 % (w/w) of a binder;
iv) 0.5 to 5 % (w/w) of disintegrant;
v) 0.25 to 3 % (w/w) of a lubricant;
vi) 0 to 2 % (w/w) of a glidant; and
vii) a total of 0 to 10 % (w/w) of one or more additional pharmaceutically acceptable excipients.

20. A pharmaceutical preparation according to one or more of claims 16 to 19, wherein the filler is mannitol , the binder is microcrystalline cellulose, the disintegrant is selected from crospovidone, carboxy starch glycolate, carboxymethylcellulose and salts and derivatives thereof, especially croscarmellose sodium, the lubricant is selected from magnesium stearate, calcium stearate, stearic acid, glycerol fatty acid esters and sodium stearyl fumarate and/or the glidant is selected from colloidal silicon dioxide and derivatives thereof.

21. A method for preparing the solid preparation according to any of claims 1 to 10, the method comprising dry granulating.

22. The method for preparing the solid preparation according to Claim 21, the method comprising:
(a) mixing 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutical acceptable salt thereof and a filler and optionally one or more further pharmaceutically acceptable excipient;
(b) dry granulating the mixture prepared by step (a) to form the solid preparation; and
(c) optionally milling.

23. A method for preparing the solid preparation according to Claim 21 or 22, wherein dry granulating is roller compacting.

24. A method for preparing a pharmaceutical preparation, which is a tablet, comprising a solid preparation according to any of claims 1 to 10, comprising
(a) conducting the method according to claim 21, 22 or 23 to form the solid preparation;
(b) mixing the solid preparation and one or more pharmaceutically acceptable excipients;
(c) tableting the mixture prepared by step (b); and
(d) optionally film coating of the tablets prepared by step (c).

25. A method for preparing a pharmaceutical preparation, which is a capsule, comprising a solid preparation according to one or more of Claims 1 to 10, comprising
(a) conducting the method according to claim 21, 22 or 23 to form the solid preparation;
(b) optionally mixing the solid preparation and one or more pharmaceutically acceptable excipient; and
(c) filling the solid preparation prepared by step (a) or the mixture prepared by step (b) into capsules.

26. The pharmaceutical preparation according to one or more of Claims 11 to 20 for use in the treatment of cancer optionally together with radiotherapy.

27. The pharmaceutical preparation for use in the treatment of cancer according to Claim 26, wherein the treatment further comprises chemotherapy.

28. The pharmaceutical preparation for use in the treatment of cancer according to Claim 26 or 27, wherein the treatment further comprises immunotherapy.

## Patentansprüche

1. Feste Zubereitung enthaltend mikronisiertes 3-(1-{3-[5-(1-Methyl-piperidin-4-yl-methoxy)-pyrimidin-2-yl]-benzyl}-6-oxo-1,6-dihydro-pyridazin-3-yl)-benzonitril oder ein pharmazeutisch unbedenkliches Salz davon und ein Füllstoff, bei der 3-(1-{3-[5-(1-Methyl-piperidin-4-yl-methoxy)-pyrimidin-2-yl]-benzyl}-6-oxo-1,6-dihydro-pyridazin-3-yl)-benzonitril oder sein pharmazeutisch unbedenkliches Salz mit 20 bis 80 % (w/w) vorliegt, bezogen auf das Gesamtgewicht der festen Zubereitung.

2. Feste Zubereitung nach Anspruch 1, bei der 3-(1-{3-[5-(1-Methyl-piperidin-4-yl-methoxy)-pyrimidin-2-yl]-benzyl}-6-oxo-1,6-dihydro-pyridazin-3-yl)-benzonitril in Form seines Sulfats, Phosphats, Mesylats, Besylats, Tosylats, Fumarats, Monohydrochlorid-monohydrats oder Maleats, vorzugsweise Monohydrochlorid-monohydrats, vorliegt.

3. Feste Zubereitung nach Anspruch 1 oder 2, bei der 3-(1-{3-[5-(1-Methylpiperidin-4-yl-methoxy)-pyrimidin-2-yl]-benzyl}-6-oxo-1,6-dihydro-pyridazin-3-yl)-benzonitril eine mittlere Teilchengröße aufweist, die durch einen d50-Wert im Bereich von 5 µm bis 80 µm, vorzugsweise von 5 µm bis 50 µm und stärker bevorzugt von 5 µm bis 25 µm gekennzeichnet ist.

4. Feste Zubereitung nach einem oder mehreren der Ansprüche 1 bis 3, bei der der Füllstoff ein Zucker, ein Zuckeralkohol oder Dicalciumphosphat ist.

5. Feste Zubereitung nach Anspruch 4, bei der der Füllstoff ein Zuckeralkohol ist, wobei der Zuckeralkohol Sorbit und/oder Mannit, vorzugsweise Mannit, ist.

6. Feste Zubereitung nach einem oder mehreren der Ansprüche 1 bis 5, bei der die feste Zubereitung ferner ein Bindemittel umfasst.

7. Feste Zubereitung nach Anspruch 6, bei der das Bindemittel Polyvinylpyrrolidon, Polyvinylacetat, ein Vinylpyrrolidon-Vinylacetat-Copolymer, Polyethylenglykol, eine Stärkepaste wie Maisstärkepaste oder ein Cellulosederivat wie Hydroxypropylmethylcellulose, Hydroxypropylcellulose oder mikrokristalline Cellulose, vorzugsweise mikrokristalline Cellulose, ist.

8. Feste Zubereitung nach einem oder mehreren der Ansprüche 1 bis 7, bei der die feste Formulierung ferner ein Schmiermittel umfasst.

9. Feste Zubereitung nach Anspruch 8, bei der das Schmiermittel Natriumstearylfumarat, Ester von Glycerin mit Fettsäuren, Stearinsäure oder pharmazeutisch unbedenkliche Salze der Stearinsäure mit zweiwertigen Kationen, vorzugsweise Magnesiumstearat, ist.

10. Feste Zubereitung nach einem oder mehreren der Ansprüche 1 bis 9, bei der die feste Zubereitung eine mittlere Teilchengröße aufweist, die durch einen d50-Wert im Bereich von 50 µm bis 1 mm, vorzugsweise von 60 µm bis 800 µm und stärker bevorzugt von 70 bis 600 µm gekennzeichnet ist.

11. Pharmazeutische Zubereitung umfassend die feste Zubereitung nach einem oder mehreren der Ansprüche 1 bis 10.

12. Pharmazeutische Zubereitung nach Anspruch 11, bei der es sich um eine pharmazeutische Zubereitung zur oralen Verabreichung handelt.

13. Pharmazeutische Zubereitung nach Anspruch 11 oder 12, bei der es sich um eine Zubereitung zur sofortigen Freisetzung handelt.

14. Pharmazeutische Zubereitung nach einem oder mehreren der Ansprüche 11 bis 13, bei der es sich um eine Kapsel handelt, welche die feste Zubereitung und gegebenenfalls einen oder mehrere pharmazeutisch unbedenkliche Hilfsstoffe umfasst.

15. Pharmazeutische Zubereitung nach Anspruch 14, bei der es sich um eine Kapsel handelt, welche 40 bis 100 % (w/w) der festen Zubereitung nach beliebigen der Ansprüche 1 bis 10 und 0 bis 60 % (w/w) mindestens eines pharmazeutisch unbedenklichen Hilfsstoffs, vorzugsweise ausgewählt aus einem Füllstoff, einem Gleitmittel, einem Sprengmittel und einem Schmiermittel, bezogen auf das Gesamtgewicht des ganzen in der Kapsel enthaltenen Materials, umfasst.

16. Pharmazeutische Zubereitung nach einem oder mehreren der Ansprüche 11 bis 13, bei der es sich um eine Tablette handelt und die zusätzlich zu den in der festen Zubereitung vorliegenden pharmazeutisch unbedenklichen Hilfsstoffen gegebenenfalls einen oder mehrere pharmazeutisch unbedenkliche Hilfsstoffe, die aus einem Füllstoff, einem Sprengmittel, einem Gleitmittel und einem Schmiermittel ausgewählt sind, umfasst.

17. Pharmazeutische Zubereitung nach Anspruch 16, bei der es sich um eine Tablette handelt, welche die feste Zubereitung nach beliebigen der Ansprüche 1 bis 10 und gegebenenfalls weitere Hilfsstoffe umfasst, wobei die Tablette, bezogen auf ihr Gesamtgewicht, umfasst:
i) 20 bis 80 % (w/w) 3-(1-{3-[5-(1-Methyl-piperidin-4-yl-methoxy)-pyrimidin-2-yl]-benzyl}-6-oxo-1,6-dihydro-pyridazin-3-yl)-benzonitril oder ein pharmazeutisch unbedenkliches Salz davon;
ii) 10 bis 70 % (w/w) eines Füllstoffs;
iii) 0 bis 20 % (w/w) eines Bindemittels;
iv) 0 bis 20 % (w/w) Sprengmittel;
v) 0 bis 5 % (w/w) eines Schmiermittels;
vi) 0 bis 7,5 % (w/w) Gleitmittel und
vii) insgesamt 0 bis 20 % (w/w) eines oder mehrerer zusätzlicher pharmazeutisch unbedenklicher Hilfsstoffe.

18. Pharmazeutische Zubereitung nach Anspruch 16 oder 17, bei der es sich um eine Tablette handelt, welche die feste Zubereitung nach beliebigen der Ansprüche 1 bis 10 und gegebenenfalls weitere Hilfsstoffe umfasst, wobei die Tablette, bezogen auf ihr Gesamtgewicht, umfasst:
i) 30 bis 70 % (w/w) 3-(1-{3-[5-(1-Methyl-piperidin-4-yl-methoxy)-pyrimidin-2-yl]-benzyl}-6-oxo-1,6-dihydro-pyridazin-3-yl)-benzonitril oder ein pharmazeutisch unbedenkliches Salz davon;
ii) 20 bis 60 % (w/w) eines Füllstoffs;
iii) 0 bis 10 % (w/w) eines Bindemittels;
iv) 0,25 bis 10 % (w/w) Sprengmittel;
v) 0 bis 4 % (w/w) eines Schmiermittels;
vi) 0 bis 5 % (w/w) eines Gleitmittels und
vii) insgesamt 0 bis 10 % (w/w) eines oder mehrerer zusätzlicher pharmazeutisch unbedenklicher Hilfsstoffe.

19. Pharmazeutische Zubereitung nach Anspruch 16, 17 oder 18, bei der es sich um eine Tablette handelt, die umfasst:
i) 35 bis 60 % (w/w) 3-(1-{3-[5-(1-Methyl-piperidin-4-yl-methoxy)-pyrimidin-2-yl]-benzyl}-6-oxo-1,6-dihydro-pyridazin-3-yl)-benzonitril oder ein pharmazeutisch unbedenkliches Salz davon;
ii) 40 bis 60 % (w/w) eines Füllstoffs;
iii) 0 bis 5 % (w/w) eines Bindemittels;
iv) 0,5 bis 5 % (w/w) Sprengmittel;
v) 0,25 bis 3 % (w/w) eines Schmiermittels;
vi) 0 bis 2 % (w/w) eines Gleitmittels und
vii) insgesamt 0 bis 10 % (w/w) eines oder mehrerer zusätzlicher pharmazeutisch unbedenklicher Hilfsstoffe.

20. Pharmazeutische Zubereitung nach einem oder mehreren der Ansprüche 16 bis 19, bei der der Füllstoff Mannit ist, das Bindemittel mikrokristalline Cellulose ist, das Sprengmittel aus Crospovidon, Carboxystärkeglykolat, Carboxymethylcellulose und Salzen und Derivaten davon, insbesondere Croscarmellose-Natrium, ausgewählt ist, das Schmiermittel aus Magnesiumstearat, Calciumstearat, Stearinsäure, Glycerinfettsäureestern und Natriumstearylfumarat ausgewählt ist und/oder das Gleitmittel aus kolloidalem Siliciumdioxid und Derivaten davon ausgewählt ist.

21. Verfahren zur Herstellung der festen Zubereitung nach beliebigen der Ansprüche 1 bis 10, welches Trockengranulierung umfasst.

22. Verfahren zur Herstellung der festen Zubereitung nach Anspruch 21, welches umfasst:
(a) Mischen von 3-(1-{3-[5-(1-Methyl-piperidin-4-yl-methoxy)-pyrimidin-2-yl]-benzyl}-6-oxo-1,6-dihydro-pyridazin-3-yl)-benzonitril oder einem pharmazeutisch unbedenklichen Salz davon und einem Füllstoff und gegebenenfalls einem oder mehreren weiteren pharmazeutisch unbedenklichen Hilfsstoffen;
(b) Trockengranulieren der durch Schritt (a) hergestellten Mischung zur Bildung der festen Zubereitung; und
(c) gegebenenfalls Mahlen.

23. Verfahren zur Herstellung der festen Zubereitung nach Anspruch 21 oder 22, bei dem die Trockengranulierung Walzenkompaktierung ist.

24. Verfahren zur Herstellung einer pharmazeutischen Zubereitung, bei der es sich um eine Tablette handelt, welche eine feste Zubereitung nach beliebigen der Ansprüche 1 bis 10 umfasst, umfassend
(a) Durchführen des Verfahrens nach Anspruch 21, 22 oder 23 zur Bildung der festen Zubereitung;
(b) Mischen der festen Zubereitung und eines oder mehrerer pharmazeutisch unbedenklicher Hilfsstoffe;
(c) Tablettieren der durch Schritt (b) hergestellten Mischung; und
(d) gegebenenfalls Filmbeschichten der durch Schritt (c) hergestellten Tabletten.

25. Verfahren zur Herstellung einer pharmazeutischen Zubereitung, bei der es sich um eine Kapsel handelt, welche eine feste Zubereitung nach einem oder mehreren der Ansprüche 1 bis 10 umfasst, umfassend
(a) Durchführen des Verfahrens nach Anspruch 21, 22 oder 23 zur Bildung der festen Zubereitung;
(b) gegebenenfalls Mischen der festen Zubereitung und eines oder mehrerer pharmazeutisch unbedenklicher Hilfsstoffe; und
(c) Einfüllen der durch Schritt (a) hergestellten festen Zubereitung oder der durch Schritt (b) hergestellten Mischung in Kapseln.

26. Pharmazeutische Zubereitung nach einem oder mehreren der Ansprüche 11 bis 20 zur Verwendung bei der Behandlung von Krebs, gegebenenfalls zusammen mit Radiotherapie.

27. Pharmazeutische Zubereitung zur Verwendung bei der Behandlung von Krebs nach Anspruch 26, bei der die Behandlung ferner Chemotherapie umfasst.

28. Pharmazeutische Zubereitung zur Verwendung bei der Behandlung von Krebs nach Anspruch 26 oder 27, bei der die Behandlung ferner Immuntherapie umfasst.

## Revendications

1. Préparation solide comprenant du 3-(1-{3-[5-(1-méthylpipéridin-4-ylméthoxy)-pyrimidin-2-yl]benzyl}-6-oxo-1,6-dihydropyridazin-3-yl)benzonitrile micronisé ou un sel pharmaceutiquement acceptable de celui-ci et une charge, dans laquelle le 3-(1-{3-[5-(1-méthylpipéridin-4-ylméthoxy)pyrimidin-2-yl]benzyl}-6-oxo-1,6-dihydropyridazin-3-yl)benzonitrile ou son sel pharmaceutiquement acceptable est présent de 20 à 80% (p/p) sur la base du poids total de la préparation solide.

2. Préparation solide selon la revendication 1, dans laquelle le 3-(1-{3-[5-(1-méthylpipéridin-4-ylméthoxy)pyrimidin-2-yl]benzyl}-6-oxo-1,6-dihydropyrida-zin-3-yl)benzonitrile est présent sous la forme de son sulfate, phosphate, mésylate, bésylate, tosylate, fumarate, monochlorhydrate monohydraté ou maléate, préférablement monochlorhydrate monohydraté.

3. Préparation solide selon la revendication 1 ou 2, dans laquelle le 3-(1-{3-[5-(1-méthylpipéridin-4-ylméthoxy)pyrimidin-2-yl]benzyl}-6-oxo-1,6-dihydropy-ridazin-3-yl)benzonitrile présente une taille moyenne de particules qui est **caractérisée par** une valeur d50 dans la plage allant de 5 µm à 80 µm, préférablement de 5 µm à 50 µm et plus préférablement de 5 µm à 25 µm.

4. Préparation solide selon l'une ou plusieurs parmi les revendications 1 à 3, dans laquelle la charge est un sucre, un alcool de sucre ou un phosphate dicalcique.

5. Préparation solide selon la revendication 4, dans laquelle la charge est un alcool de sucre, où l'alcool de sucre est le sorbitol et/ou le mannitol, préférablement le mannitol.

6. Préparation solide selon l'une ou plusieurs parmi les revendications 1 à 5, la préparation solide comprenant en outre un liant.

7. Préparation solide selon la revendication 6, dans laquelle le liant est la polyvinylpyrrolidone, l'acétate de polyvinyle, un copolymère de vinylpyrrolidone-acétate de vinyle, le polyéthylène glycol, une pâte d'amidon telle que la pâte d'amidon de maïs, ou un dérivé de cellulose tel que l'hydroxypropyl méthylcellulose, l'hydroxypropylcellulose ou la cellulose microcristalline, préférablement la cellulose microcristalline.

8. Préparation solide selon l'une ou plusieurs parmi les revendications 1 à 7, dans laquelle la formulation solide comprend en outre un lubrifiant.

9. Préparation solide selon la revendication 8, dans laquelle le lubrifiant est le stéarylfumarate de sodium, les esters d'acides gras et de glycérol, l'acide stéarique, ou des sels pharmaceutiquement acceptables d'acide stéarique et de cations divalents, préférablement le stéarate de magnésium.

10. Préparation solide selon l'une ou plusieurs parmi les revendications 1 à 9, la préparation solide présentant une taille moyenne de particules qui est **caractérisée par** une valeur d50 dans la plage allant de 50 µm à 1 mm, préférablement de 60 µm à 800 µm et plus préférablement de 70 à 600 µm.

11. Préparation pharmaceutique comprenant la préparation solide selon l'une ou plusieurs parmi les revendications 1 à 10.

12. Préparation pharmaceutique selon la revendication 11, qui est une préparation pharmaceutique destinée à une administration orale.

13. Préparation pharmaceutique selon la revendication 11 ou 12, qui est une préparation à libération immédiate.

14. Préparation pharmaceutique selon l'une ou plusieurs parmi les revendications 11 à 13, qui est une capsule comprenant la préparation solide et éventuellement un ou plusieurs excipients pharmaceutiquement acceptables.

15. Préparation pharmaceutique selon la revendication 14, qui est une capsule, qui contient de 40 à 100% (p/p) de la préparation solide selon l'une quelconque des revendications 1 à 10 ; et de 0 à 60% (p/p) d'au moins un excipient pharmaceutiquement acceptable, choisi de préférence parmi une charge, un agent de glissement, un agent délitant et un lubrifiant, sur la base du poids total de tous les matériaux contenus dans la capsule.

16. Préparation pharmaceutique selon l'une ou plusieurs parmi les revendications 11 à 13, qui est un comprimé et qui, outre les excipients pharmaceuti-quement acceptables présents dans la préparation solide, comprend éventuellement un ou plusieurs excipients pharmaceutiquement acceptables choisis parmi une charge, un agent délitant, un agent de glissement et un lubrifiant.

17. Préparation pharmaceutique selon la revendication 16, qui est un comprimé comprenant la préparation solide selon l'une quelconque des revendications 1 à 10 et éventuellement d'autres excipients, lequel comprimé, sur la base de son poids total, comprend :
i) de 20 à 80% (p/p) de 3-(1-{3-[5-(1-méthylpipéridin-4-ylméthoxy)-pyrimidin-2-yl]benzyl}-6-oxo-1,6-dihydropyridazin-3-yl)benzonitrile ou d'un sel pharmaceutiquement acceptable de celui-ci ;
ii) de 10 à 70% (p/p) d'une charge ;
iii) de 0 à 20% (p/p) d'un liant ;
iv) de 0 à 20% (p/p) d'un agent délitant ;
v) de 0 à 5% (p/p) d'un lubrifiant ;
vi) de 0 à 7,5% (p/p) d'un agent de glissement ; et
vii) un total de 0 à 20% (p/p) d'un ou plusieurs autres excipients pharmaceutiquement acceptables.

18. Préparation pharmaceutique selon la revendication 16 ou 17, qui est un comprimé comprenant la préparation solide selon l'une quelconque des revendications 1 à 10 et éventuellement d'autres excipients, lequel comprimé, sur la base de son poids total, comprend :
i) de 30 à 70% (p/p) de 3-(1-{3-[5-(1-méthylpipéridin-4-ylméthoxy)-pyrimidin-2-yl]benzyl}-6-oxo-1,6-dihydropyridazin-3-yl)benzonitrile ou d'un sel pharmaceutiquement acceptable de celui-ci ;
ii) de 20 à 60% (p/p) d'une charge ;
iii) de 0 à 10% (p/p) d'un liant ;
iv) de 0,25 à 10% (p/p) d'un agent délitant ;
v) de 0 à 4% (p/p) d'un lubrifiant ;
vi) de 0 à 5% (p/p) d'un agent de glissement ; et
vii) un total de 0 à 10% (p/p) d'un ou plusieurs autres excipients pharmaceutiquement acceptables.

19. Préparation pharmaceutique selon la revendication 16, 17 ou 18, qui est un comprimé comprenant:
i) de 35 à 60% (p/p) de 3-(1-{3-[5-(1-méthylpipéridin-4-ylméthoxy)-pyrimidin-2-yl]benzyl}-6-oxo-1,6-dihydropyridazin-3-yl)benzonitrile ou d'un sel pharmaceutiquement acceptable de celui-ci ;
ii) de 40 à 60% (p/p) d'une charge ;
iii) de 0 à 5% (p/p) d'un liant ;
iv) de 0,5 à 5% (p/p) d'un agent délitant ;
v) de 0,25 à 3% (p/p) d'un lubrifiant ;
vi) de 0 à 2% (p/p) d'un agent de glissement ; et
vii) un total de 0 à 10% (p/p) d'un ou plusieurs autres excipients pharmaceutiquement acceptables.

20. Préparation pharmaceutique selon l'une ou plusieurs parmi les revendications 16 à 19, dans laquelle la charge est le mannitol, le liant est la cellulose microcristalline, l'agent délitant est choisi parmi la crospovidone, le glycolate de carboxy-amidon, la carboxyméthylcellulose et les sels et dérivés de celle-ci, notamment la croscarmellose de sodium, le lubrifiant est choisi parmi le stéarate de magnésium, le stéarate de calcium, l'acide stéarique, les esters d'acide gras et de glycérol et le stéarylfumarate de sodium et/ou l'agent de glissement est choisi parmi le dioxyde de silicium colloïdal et des dérivés de celui-ci.

21. Méthode de préparation de la préparation solide selon l'une quelconque des revendications 1 à 10, la méthode comprenant une granulation à sec.

22. Méthode de préparation de la préparation solide selon la revendication 21, la méthode comprenant :
(a) le mélange de 3-(1-{3-[5-(1-méthylpipéridin-4-ylméthoxy)pyrimidin-2-yl]benzyl}-6-oxo-1,6-dihydropyridazin-3-yl)benzonitrile ou d'un sel pharmaceutiquement acceptable de celui-ci et d'une charge et éventuellement d'un ou plusieurs autres excipients pharmaceutiquement acceptables ;
(b) la granulation à sec du mélange préparé à l'étape (a) afin de former la préparation solide ; et
(c) éventuellement un broyage.

23. Méthode de préparation de la préparation solide selon la revendication 21 ou 22, dans laquelle la granulation à sec est un compactage par rouleaux.

24. Méthode de préparation d'une préparation pharmaceutique, qui est un comprimé, comprenant une préparation solide selon l'une quelconque des revendications 1 à 10, comprenant
(a) la mise en oeuvre de la méthode selon la revendication 21, 22 ou 23, afin de former la préparation solide ;
(b) le mélange de la préparation solide et d'un ou plusieurs excipients pharmaceutiquement acceptables ;
(c) la mise en comprimés du mélange préparé à l'étape (b) ; et
(d) éventuellement le pelliculage des comprimés préparés à l'étape (c).

25. Méthode de préparation d'une préparation pharmaceutique, qui est une capsule, comprenant une préparation solide selon l'une ou plusieurs parmi les revendications 1 à 10, comprenant
(a) la mise en oeuvre de la méthode selon la revendication 21, 22 ou 23, afin de former la préparation solide ;
(b) éventuellement le mélange de la préparation solide et d'un ou plusieurs excipients pharmaceutiquement acceptables ; et
(c) le chargement de la préparation solide préparée à l'étape (a) ou du mélange préparé à l'étape (b) dans des capsules.

26. Préparation pharmaceutique selon l'une ou plusieurs parmi les revendications 11 à 20, pour une utilisation dans le traitement d'un cancer, éventuellement conjointement avec une radiothérapie.

27. Préparation pharmaceutique pour une utilisation dans le traitement d'un cancer selon la revendication 26, le traitement comprenant en outre une chimiothérapie.

28. Préparation pharmaceutique pour une utilisation dans le traitement d'un cancer selon la revendication 26 ou 27, le traitement comprenant en outre une immunothérapie.
